# NEUE EUROPÄISCHE PATENTSCHRIFT

(11) **EP 2 111 416 B2**
(45) Veröffentlichungstag und Bekanntmachung des Hinweises auf die Entscheidung über den Einspruch: **30.03.2022**
(45) Hinweis auf die Patenterteilung: 15.05.2019
(21) Anmeldenummer: 08707897.8
(22) Anmeldetag: 15.01.2008
(51) Int. Cl.: C08B 37/00, C07H 23/00, C08B 37/16, C08B 37/02, A61K 33/26, A61K 31/715

(54) **EISEN-KOHLENHYDRAT-KOMPLEX-VERBINDUNGEN**
IRON-HYDROCARBON COMPLEX COMPOUNDS
COMPOSÉS COMPLEXES DE FER/GLUCIDES

(30) Priorität: 19.01.2007 EP 07100803
(43) Veröffentlichungstag der Anmeldung: 28.10.2009
(73) Patentinhaber: VIFOR (INTERNATIONAL) AG, 9001 St. Gallen (CH)
(72) Erfinder: REIM, Stefan, CH-9014 St. Gallen (CH); GEISSER, Peter, CH-9014 St. Gallen (CH)
(74) Vertreter: Gille Hrabal Partnerschaftsgesellschaft mbB Patentanwälte
(86) Internationale Anmeldenummer: PCT/EP2008/050387
(87) Internationale Veröffentlichungsnummer: WO 2008/087135

(56) Entgegenhaltungen:
- EP-A2- 0 044 050
- WO-A-2004/037865
- WO-A-2005/000210
- WO-A-2005/116046
- BR-A- PI0 303 685
- DE-A1- 3 844 065
- GB-A- 1 111 929
- US-B1- 6 461 651
- DATABASE WPI Week 200371 Derwent Publications Ltd., London, GB; AN 2003-754172 XP002449716 "Method for preparing iron-containing medicinal agent" & RU 2 198 665 C1 (A BIO CO LTD) 20. Februar 2003 (2003-02-20) & DATABASE CA CHEMICAL ABSTRACTS SERVICE, COLUMBUS, OHIO, US; gefunden im STN Database accession no. 139:169280

## Beschreibung

Die Erfindung betrifft Eisen-Kohlenhydrat-Komplex-Verbindungen, die neben Eisen(III), Eisen(II) enthalten, Verfahren zu ihrer Herstellung, sie enthaltende Arzneimittel, sowie deren Verwendung zur Behandlung von Eisenmangelanämien.

Durch Eisenmangel bedingte Anämien werden nach dem Stand der Technik insbesondere durch parenterale Verabreichung von Eisen(III) enthaltenen Arzneimitteln und insbesondere durch orale Verabreichung von Eisen(II)- oder Eisen(III)-enthaltenen Arzneimitteln therapiert oder prophylaktisch behandelt. Eine parenterale Verabreichung von Eisen(II) erfolgt also nicht.

Ein in der Praxis häufig verwendetes Präparat ist ein wasserlöslicher Eisen(III)-Hydroxid-Saccharose-Komplex (Danielson, Salomonson, Derendorf, Geisser, Drug Res., Vol., 46 : 615 - 621, 1996), der sich besonders zur parenteralen Verabreichung eignet.

Die WO2004/037865 A1 offenbart einen wasserlöslichen Eisen(III)-Hydroxid-Kohlenhydrat-Komplex, der vorwiegend parenteral, aber auch oral, anwendbar ist. Es handelt sich um einen Komplex aus Eisen(III) mit Maltodextrinen mit einem Dextrose-Äquivalent von 5 bis 20, wobei das Molekulargewicht des Komplexes bei 80 bis 400 kDa liegt.

Ein weiteres erfolgreich angewendetes oral verabreichbares Präparat basiert auf einem Eisen(III)-Hydroxid-Polymaltose-Komplex mit einem Molekulargewicht von etwa 50 kDa, welches im Handel als Maltofer^{®} erhältlich ist,

Auf dem Markt befindliche orale Eisen(II)-Präparate sind insbesondere Eisen(II)-fumarat, -sulfat und -glycolat.

Klinische Studien haben gezeigt, dass Eisen(II)-Verbindungen im allgemeinen rascher absorbiert werden. Es gibt Theorien, wonach Eisen(III) bei oraler Aufnahme über einen zweiwertigen Zwischenzustand absorbiert wird [Hentze, M. W., Muckenthaler, M. U. und Andrews N. C. (2004) Balancing acts: Molecular Control of Mammalian Iron Metabolism, Cell, 117, 285-297]. Eisen(II) kann auf Grund der hohen Toxizität jedoch nicht parenteral verabreicht werden. Bei oraler Verabreichung führt es zu erhöhten Nebenwirkungen. Die orale Verabreichung von Eisen(II) ist daher nicht bevorzugt. Der Erfindung lag daher die Aufgabe zugrunde, ein verbessertes Arzneimittel zur Verfügung zu stellen, durch das Eisen insbesondere bei oraler Verabreichung besonders gut vom Körper aufgenommen wird. Insbesondere soll das Eisen möglichst zügig resorbiert werden und besser verträglich sein als reine Eisen(II)-Präparate.

Die Aufgabe wird erfindungsgemäß durch die Bereitstellung einer Eisen-Kohlenhydrat-Komplex-Verbindung gelöst, welche einen hohen Anteil an Eisen(II) bei tolerierbar geringer Toxizität aufweist.

Die Erfindung ist in den Ansprüchen definiert.

Die Erfindung stellt somit eine Eisen-Kohlenhydrat-Komplex-Verbindung bereit, wie im Anspruch 1 definiert, nachfolgend auch die "erfindungsgemäße Eisen-Kohlenhydrat-Komplex-Verbindung.

Bei den erfindungsgemäßen Eisen-Kohlenhydrat-Komplex-Verbindungen handelt es sich insbesondere um oligo- oder polynukleare Eisenverbindungen, in denen die Eisenatome insbesondere über Sauerstoffatome und/oder Hydroxylgruppen miteinander verbunden sind und worin die Kohlenhydrate teilweise komplex und/oder über Wasserstoffbrückenbindungen gebunden vorliegen. Darüber hinaus können oxidierte Kohlenhydratmoleküle über Carboxylatgruppen komplex gebunden vorliegen, wie weiter unten beschrieben. Weiterhin können die Eisen-Kohlenhydrat-Komplex-Verbindungen auch Wasser komplex oder über Wasserstoffbrückenbindungen gebunden enthalten.

Die erfindungsgemäßen Eisen-Kohlenhydrat-Komplex-Verbindungen sind gekennzeichnet durch ihren Gehalt von Eisen(II). Dies meint, dass in der erfindungsgemäßen Eisen-Kohlenhydrat-Komplex-Verbindung ein Teil des Eisens in der Oxidationsstufe 2+ vorliegt. Das übrige Eisen liegt in der erfindungsgemäßen Eisen-Kohlenhydrat-Komplex-Verbindung praktisch ausschließlich in der Oxidationsstufe 3+, d.h. als Eisen(III) vor. Es handelt sich also um sogenannte "mixed valence"-Verbindungen, bei denen das Metall nebeneinander in mehreren Oxidationsstufen vorliegt.

Der Anteil des Eisen(II) am Gesamteisengehalt beträgt erfindungsgemäß 2 Gew.-% bis 50 Gew.-%, bezogen auf die Gesamtmenge des Eisens in der Eisen-Kohlenhydrat-Komplex-Verbindung. Vorzugsweise beträgt der Anteil von Eisen(II) am Gesamteisengehalt 3 bis 50 Gew.-%, bevorzugter 5 bis 40 Gew.-%, besonders bevorzugt 7 bis 35 Gew.-% jeweils bezogen auf die Gesamtmenge des Eisens in der Eisen-Kohlenhydrat-Komplex-Verbindung. Die Bestimmung des Eisen(II)-Gehaltes kann insbesondere durch eine titrimetrische Bestimmung erfolgen (siehe zum Beispiel: Jander Jahr, Maßanalyse 15. Auflage, Verlag Walter de Gruyter, 1989). Dabei wird zunächst Gesamteisen unter Einsatz von H₂O₂ und anschließend Eisen(III) ohne Verwendung von H₂O₂ bestimmt und der Gehalt an Eisen(II) durch Differenzbildung ermittelt.

Der Anteil des gesamten Eisens am Gewicht der Eisen-Kohlenhydrat-Komplex-Verbindung beträgt vorzugsweise 5 bis 40 Gew.-%, bevorzugt 10 bis 30 Gew,-%.

Der Anteil des Kohlenhydrates (bzw. der Kohlenhydrate) am Gewicht der Komplex-Verbindung beträgt in einer bevorzugten Ausführungsform 10 bis 80 Gew.-%, bevorzugt 20 bis 70 Gew.-%, besonders bevorzugt 35 bis 65 Gew.-%.

Die im Rahmen der vorliegenden Erfindung auf das Gewicht der Eisen-Kohlenhydrat-Komplex-Verbindung bezogenen Mengenangaben, beziehen sich stets auf das Gesamtgewicht der erfindungsgemäßen Eisen-Kohlenhydrat-Komplex-Verbindung einschließlich beispielweise des gegebenenfalls aus der Herstellung resultierenden Wassergehalts, wie unten stehend angegeben.

Neben Eisen(III), Eisen(II) und einem oder mehreren Kohlenhydraten, enthält die erfindungsgemäße Eisen-Kohlenhydrat-Komplex-Verbindung Hydroxy-Gruppen (im allgemeinen als OH⁻ bezeichnet) Oxo-Gruppen (im allgemeinen als O²⁻ bezeichnet) gegebenenfalls weitere Anionen sowie Wasser. Die ionogene Schreibweise als OH⁻ oder O²⁻ schließt dabei selbstverständlich nicht aus, dass diese Gruppen bei ihrer Bindung an Eisen-Kationen mehr oder weniger kovalente Bindungsanteile aufweisen, Dies ist dem Fachmann wohl bekannt.

Die erfindungsgemäßen Eisen-Kohlenhydrat-Komplex-Verbindungen können neben den Kohlenhydraten auch andere Liganden enthalten, beispielsweise Carbonsäuren wie Gluconsäure, Milchsäure etc.

Der Anteil an Wasser in der erfindungsgemäßen Eisen-Kohlenhydrat-Komplex-Verbindung kann dabei je nach Trocknungsbedingungen zweckmäßig bis zu 10 Gew.-% betragen. Bevorzugt liegt der Wassergehalt bei 2 bis 8 Gew.-%,

Eine erfindungsgemäße Eisen-Kohlenhydrat-Komplex-Verbindung hat beispielsweise folgende Zusammensetzung:
- 5 bis 40 Gew-% Eisen, wovon bevorzugt 3 bis 50 Gew.-%, bevorzugter 5 bis 40 Gew.-%, bezogen auf die Gesamt-Eisenmenge, in der Form von Eisen(II) vorliegen,
- 10 bis 80 Gew,-%, bevorzugt 20 bis 70 Gew.-%, besonders bevorzugt 35 bis 65 Gew.-% ein oder mehrere Kohlenhydrate,
- Rest: Sauerstoff und Wasserstoff in gebundener Form (außer im Kohlenhydrat) sowie gegebenenfalls weitere Elemente.

Wie zuvor dargelegt, liegen die Elemente Sauerstoff und Wasserstoff insbesondere als Hydroxy-Gruppen, Oxo-Gruppen und gegebenenfalls Wasser vor. Weitere Elemente, neben Eisen, Kohlenstoff, Sauerstoff, Wasserstoff und Stickstoff können beispielsweise durch den Eintrag aus dem bei der Herstellung verwendeten Eisen(III)-Salz sowie gegebenenfalls bei der Herstellung verwendeter Säuren und/oder Basen resultieren. So handelt es sich beispielsweise um Chlor (beispielsweise aus Cl⁻), Schwefel beispielsweise aus Sulfat (SO₄²⁻), Stickstoff beispielsweise aus Nitrat (NO₃⁻) und Alkali- und Erdalkalimetalle aus den verwendeten Basen, wie Alkali- und Erdalkalimetall-Hydroxiden, -Carbonaten oder -Bicarbonaten etc. Der Anteil der weiteren Elemente liegt im allgemeinen unter 15 bevorzugter unter 10 Gew.-%, bezogen auf das Gewicht der erfindungsgemäßen Eisen-Kohlenhydrat-Komplex-Verbindung.

Bevorzugt ist die Zusammensetzung:
- 10 bis 30 Gew-% Eisen, wovon bevorzugt 5 bis 40 Gew.-% bezogen auf die Gesamt-Eisenmenge in der Form von Eisen(II) vorliegen,
- 20 bis 70 Gew.-% ein oder mehrere Kohlenhydrate,
- Rest: Sauerstoff und Wasserstoff in gebundener Form (außer in den Kohlenhydraten) sowie gegebenenfalls weitere Elemente, wie zuvor erläutert.

In einer besonderen Ausführungsform der Erfindung liegt das gewichtsmittlere Molekulargewicht der erfindungsgemäßen Eisen-Kohlenhydrat-Komplex-Verbindung bei 10 bis 80 kDa, bevorzugt 12 bis 65 kDa, besonders bevorzugt 15 bis 60 kDa. Die Bestimmung des gewichtsmittleren Molekulargewichts erfolgt dabei durch Gelpermeationschromatographie gegen Pullulan als Standard (beispielsweise wie von Geisser et al. in Arzneim. Forsch/Drug Res. 42 (II), 12, 1439 - 1452 (1992), Absatz 2.2.5. beschrieben).

Die erfindungsgemäße Eisen-Kohlenhydrat-Komplex-Verbindung enthält ein oder mehrere Kohlenhydrate, wie im Anspruch 1 definiert welche beispielweise komplex und/oder über Wasserstoffbrückenbindungen an Eisen oder Eisen-haltige Partialstrukturen gebunden in den erfindungsgemäßen Verbindungen vorliegen. Die Eisen-Kohlenhydrat-Komplex-Verbindung enthält mindestens ein Kohlenhydrat ausgewählt aus unvollständig hydrolysierten Stärken, welche einen DE-Wert zwischen 0 und 100 aufweisen, Maltodextrinen mit einem DE-Wert von 3 bis 50, Maltosesirupen mit einem DE-Wert von 3 bis 50, Glukosesirupen mit einem DE-Wert von mehr als 50 und Cyclodextrinen.

Weiterhin schließt der Begriff der Kohlenhydrate erfindungsgemäß auch die Kohlenhydrat-Liganden ein, die sich aus dem erfindungsgemäß bevorzugten Herstellverfahren der Umsetzung von Eisen(III)-Salzen mit Kohlenhydraten unter Oxidation der Kohlenhydrate und Reduktion des Eisen(III) unter Bildung von Eisen(II) im Sinne einer Redox-Reaktion bilden. Bei dieser Redox-Reaktion erfolgt im allgemeinen eine Oxidation der Aldehyd- und/oder Ketogruppen (nach Umlagerung im alkalischen Milieu) der Kohlenhydrate zu Carboxylgruppen, welche beispielsweise auch den bekannten Nachweis-Verfahren von Kohlehydraten zugrundeliegt. Die sich dabei gewissermaßen in-situ-bildenden, oxidierten, Carboxylgruppenenthaltenden Kohlenhydrat-Ligand-Moleküle sind selbstverständlich auch im Umfang der Erfindung enthalten. Die Carboxylgruppen können über Wasserstoffbrückenbindungen oder anionische Carboxylatgruppen an Eisen oder Eisen-haltige Partialstrukturen, im allgemeinen direkt an Eisen gebunden vorliegen.

Die oxidierten Kohlenhydrat-Moleküle weisen somit insbesondere auch Carboxylgruppen auf, die auch zu einer stärkeren Bindung der Kohlenhydrat-Liganden an das Eisen führen.

Aus dem vorstehenden ergibt sich weiterhin, dass die erfindungsgemäß bei der Herstellung der Eisen-Kohlenhydrat-Komplex-Verbindung eingesetzten Kohlenhydrate vorzugsweise solche sind, die die Fähigkeit besitzen, Eisen(III) zu Eisen(II) zu reduzieren.

Erfindungsgemäß bevorzugt eingesetzte Kohlenhydrate oder Kohlenhydrat-Derivate schließen Maltodextrin und Maltosesirup, sowie Glucosesirupe, wie im Anspruch 1 definiert, ein.

Erfindungsgemäß verwendete Kohlenhydrate bzw. Derivate davon sind beispielsweise im Römpp-Lexikon, Biotechnologie und Gentechnik, Georg Thieme Verlag 1999, und im Lehrbuch der Lebensmittelchemie, H.-D. Belitz und W. Grosch, 4. Auflage, Springer-Verlag. beschrieben.

Der Begriff der Dextrine ist ausweislich Römpp-Lexikon, Biotechnologie und Gentechnik (ibd.) eine Sammelbezeichnung für verschiedene niedere und höhere Polymere aus D-Glucose-Einheiten der allgemeinen Formel (C₆H₁₀O₅)ₙ x H₂O, die bei unvollständiger Hydrolyse von Stärke, z.B. mit verdünnten Säuren, durch Temperatureinwirkung oder durch Einwirkung von Enzymen entstehen. Ein erfindungsgemäßes Kohlenhydrat stellt eine unvollständig hydrolysierte Stärke dar, welche einen DE-Wert zwischen 0 und 100 aufweist. Sie schließt erfindungsgemäß Maltodextrine und Maltosesirupe, sowie Glucosesirupe ein. Die erfindungsgemäß besonders bevorzugten Maltodextrine werden bevorzugt durch enzymatische Spaltung von Mais- oder Kartoffelstärke mit alpha-Amylase hergestellt. Der Hydrolysegrad wird üblicherweise in diesen Produkten durch den sogenannten DE-Wert (Dextrose-Äquivalent) angegeben. Zu diesem Zweck wird die Zunahme der Reduktionsfähigkeit einer Stärke-Lösung mit fortschreitender Hydrolyse bestimmt. Native Stärke hat den Wert DE = 0, nach vollständiger Hydrolyse zu Glucose beträgt der theoretische DE-Wert 100, und eine vollständige Spaltung zu Maltose führt zu einem DE-Wert von 52.6. Die erfindungsgemäß Maltodextrin und Maltosesirup weisen zweckmäßig einen DE-Wert von etwa 3 bis 50 auf. Der Übergang zwischen Maltodextrinen und Maltosesirupen ist dabei in der Regel fließend. Maltodextrine weisen infolge ihres geringeren Hydrolysegrades naturgemäß niedrigere DE-Werte als Maltosesirupe auf. Glucosesirupe weisen im allgemeinen höhere DE-Werte als Maltosesirupe auf (insbesondere auch mehr als 50), wobei auch hier insoweit der Übergang zwischen Maltosesirupen und Glucosesirupen in der Regel fließend ist. Im Rahmen der vorliegenden Erfindung sollen Glucosesirupe DE Werte von mehr als 50 aufweisen.

Erfindungsgemäß weisen die bevorzugt verwendeten Maltodextrine und Maltosesirupe bevorzugt DE Werte von 5 bis 45, besonders bevorzugt von 7 bis 40auf.

Erfindungsgemäß werden die Dextrose-Äquivalente insbesondere gravimetrisch bestimmt. Hierzu werden die Kohlenhydrate in wässriger Lösung mit Fehling'scher Lösung unter Sieden umgesetzt. Die Umsetzung erfolgt quantitativ, d.h. bis keine Entfärbung der Fehling'schen Lösung mehr auftritt. Das ausgefällte Kupfer(I)-Oxid wird bei 105°C bis zur Gewichtskonstanz getrocknet und gravimetrisch bestimmt. Aus den erhaltenen Werten wird der Glucosegehalt (Dextrose-Äquivalent) als % Gew./Gew. der Dextrin-Trockensubstanz berechnet. Es kann beispielsweise mit folgenden Lösungen gearbeitet werden: 25 ml Fehling'sche Lösung I, vermischt mit 25 ml Fehling'scher Lösung II; 10 ml wässrige Kohlenhydratlösung (10 % Mol/Vol) (Fehling'sche Lösung I: 34,6 g Kupfer(II)-Sulfat gelöst in 500 ml Wasser; Fehling'sche Lösung II: 173 g Kaliumnatriumtartrat und 50 g Natriumhydroxid, gelöst in 400 ml Wasser).

Es ist auch möglich, die DE-Werte nach der Methode von Lane und Eynon titrimetrisch zu bestimmen (ISO 5377 - 1981 (E)), was in erster Näherung zu vergleichbaren Ergebnissen führt.

Das zahlenmittlere Molekulargewicht der bevorzugt verwendeten Kohlenhydrate liegt zweckmäßig bei bis zu etwa 50000.

Gegenstand der Erfindung ist auch ein Verfahren zur Herstellung der erfindungsgemäßen Eisen-Kohlenhydrat-Komplex-Verbindung, wie im Anspruch 9 definiert.

Erfindungsgemäß können in Schritt b) auch Gemische von Eisen(II)- und Eisen(III)-Salzen eingesetzt werden. Dabei kann auf den Einsatz reduzierender Kohlenhydrate verzichtet werden. Weiterhin ist es erfindungsgemäß möglich, bei der Herstellung der Eisen-Kohlenhydrat-Komplex-Verbindungen auch zusätzliche Reduktionsmittel, wie z. B. Vitamin C, Dihydroflavone oder Hyperoxide zuzusetzen, welche eine Reduktion des Eisen(III) zu Eisen(II) bewirken.

In einer bevorzugten Ausführungsform des erfindungsgemäßen Verfahrens erfolgt nach dem Abkühlen der wässrigen Lösung oder Suspension in Schritt d) der Schritt d') der Einstellung des pH-Werts der wässrigen Lösung oder Suspension auf einen physiologisch verträglichen Wert von bevorzugt etwa 5 bis 9.

Gegebenenfalls während der Umsetzung gebildete Feststoffe, werden insbesondere nach Schritt d') abgetrennt, wonach der Eisenkomplex ausgefällt und isoliert werden kann.

Die Zugabe des Eisen(III)-Salzes (oder des Eisen(II)-Salzes oder eines Gemisches von Eisen(III)- und Eisen(II)-Salzen) in Schritt b) erfolgt beispielsweise durch Zutropfen einer Lösung oder Suspension unter Rühren. Als Eisen(III)-Salze (oder Eisen(II)-Salze) können bevorzugt wasserlösliche Salze anorganischer oder organischer Säuren oder Mischungen davon verwendet werden, wie Halogenide, z.B. Chloride oder Sulfate. Auch Eisenhydroxide können unter entsprechenden Bedingungen eingesetzt werden. Bevorzugt werden physiologisch unbedenkliche Salze verwendet. Besonders bevorzugt wird eine wässrige Lösung von Eisen(III)-chlorid verwendet, bevorzugt zusammen mit einem reduzierende Eigenschaften aufweisenden Kohlenhydrat. Anwendbar sind auch Fe(III)-sulfat-Lösungen und Mischungen von Eisen(III)-salz-Lösungen.

Die Zugabe des Eisen(III)-Salzes (oder des Eisen(II)-Salzes oder eines Gemisches von Eisen(II)- und Eisen(II)-Salzen) erfolgt erfindungsgemäß zweckmäßig bei pH-Werten von 7 bis 13, bevorzugt bei einem pH-Wert von 9 bis 12. Um diese pH-Werte zu erzielen und im Verlauf der Reaktion konstant zu halten, werden zweckmäßig Basen zugesetzt, wie insbesondere Alkali- oder Erdalkalihydroxide, wie Natrium-, Kalium-, Calcium- und Magnesiumhydroxid, besonders bevorzugt Natriumhydroxid oder auch Alkali- oder Erdalkalicarbonate oder -bicarbonate. Beispielsweise kann das Verfahren so durchgeführt werden, dass eine wässrige Kohlenhydratlösung auf die gewünschte Temperatur von beispielsweise 50 bis 70°C erwärmt wird und Base und Eisen(III)-Salzlösung so zugetropft werden, dass der pH-Wert konstant (beispielsweise mit einer Abweichung von maximal einer, bevorzugt 0,5 pH-Werteinheiten) gehalten wird, und wahlweise auch die Temperatur im Wesentlichen konstant gehalten wird. Bei diesem pH-Wert reagiert das Eisen(III)-Salz (oder das Eisen(II)-Salzoder ein Gemische von Eisen(III)- und Eisen(II)-Salz) im wesentlichen unter Bildung von Eisen(III) (oder Eisen(II))-Hydroxid-Bindungen. Gleichzeitig findet auch bereits Komplexbildung mit dem Kohlenhydrat statt.

Nach beendeter Zugabe der Eisen(III)-Salzlösung (oder der Eisen(II)-Salzlösung oder eines Gemisches von Eisen(III)- und Eisen(II)-Salzlösungen) sowie der Basen-Lösung wird die erhaltene Lösung oder Suspension aufgeheizt. Es findet eine weitere Reaktion statt, in der bevorzugt ein Teil des Eisen(III) durch in dem eingesetzten Kohlenhydrat, enthaltene Aldehydgruppen zu Eisen(II) reduziert wird. Das Erhitzen der Lösung erfolgt dabei vorzugsweise auf Temperaturen oberhalb 80 °C, bevorzugt oberhalb 90 °C besonders bevorzugt am Siedepunkt von Wasser (100° bei Normaldruck). Die Wärmebehandlung des Schritts c) erfolgt zweckmäßig für mindestens 30 min. Länger als 5 h dauert die Wärmebehandlung im allgemeinen nicht. Anschließend wird die Lösung abgekühlt, vorzugsweise auf 0 °C bis 30 °C, insbesondere 25 °C (Raumtemperatur).

Nach erfolgter Umsetzung wird die erhaltene Lösung oder Suspension abgekühlt und gegebenenfalls verdünnt. Nach dem Abkühlen wird der pH bevorzugt auf einen physiologisch verträglichen Wert zwischen 5 und 9, bevorzugt 5.5 und 8.5 eingestellt. Als Säuren können anorganische oder organische Säuren oder Gemische davon, insbesondere Halogenwasserstoffsäuren, wie Chlorwasserstoff bzw. wässrige Salzsäure, oder Schwefelsäure eingesetzt werden. Anschließend können möglicherweise vorhandene Feststoffe und Verunreinigungen abgetrennt werden, beispielsweise durch Filtration oder Zentrifugation.

Unter den vorstehend erwähnten Bedingungen können die erfindungsgemäßen Eisen-Kohienhydrat-Kompiex-Verbindung mit ihrem Gehalt von 2 bis 50 Gew.-% Eisen(II) bezogen auf die Eisenmenge erhalten werden.

Die erfindungsgemäßen Eisen-Kohlenhydrat-Komplex-Verbindungen sind im allgemeinen in Wasser gut löslich. Das bedeutet erfindungsgemäß, dass sich in 100 g Wasser bei 25° bevorzugt mehr als 30 g, bevorzugter mehr als 35 g, besonders bevorzugt mehr als 40 g der erfindungsgemäßen Eisen-Kohlenhydrat-Komplex-Verbindung lösen. Maximal beträgt die Löslichkeit beispielsweise etwa 100 g bis 120 g jeweils pro 100 g Wasser bei 25°C.

Die erfindungsgemäß bevorzugt erhaltenen Lösungen der Eisen-Kohlenhydrat-Komplex-Verbindungen können direkt zur Herstellung von Arzneimitteln verwendet werden. Die Lösungen werden dazu durch Umkehrosmose oder Dialyse gereinigt. Die Reinigung kann insbesondere zur Entfernung von Salzen dienen. Es ist aber auch möglich, die Eisen(III)(II)-Kohlenhydrat-Komplex-Verbindungen zunächst aus der Lösung zu isolieren, beispielsweise durch Ausfällen mit einem Alkohol, wie einem Alkanol, beispielsweise Ethanol oder Propanol. Der so erhaltene erfindungsgemäße Eisenkomplex kann zur weiteren Reinigung zusätzlich nachbehandelt werden, beispielsweise indem er mit Ethanol gemischt, filtriert und vakuumgetrocknet wird. Die Isolierung kann auch durch Sprühtrocknung nach Umkehrosmose oder Dialyse der Eisen-Kohlenhydrat-Komplex-Verbindungenthaltenden Lösung erfolgen.

Gegenstand der Erfindung ist auch ein Arzneimittel, enthaltend eine erfindungsgemäße Eisen-Kohlenhydrat-Komplex-Verbindung wie in den Ansprüchen 1-8 definiert. Aus den erfindungsgemäßen Eisen-Kohlenhydrat-Komplex-Verbindung lassen sich insbesondere sterile wässrige Lösungen herstellen.

Die erfindungsgemäßen Lösungen sind insbesondere zur oralen Applikation geeignet, sie können jedoch auch parenteral für Injektionen oder Infusionen, beispielsweise intravenös oder intramuskulär, eingesetzt werden.

Die Herstellung von parenteral verabreichbaren Lösungen kann in üblicher Weise, gegebenenfalls unter Mitverwendung von für parenterale Lösungen üblichen Zusätzen erfolgen. Die Lösungen können so formuliert werden, dass sie als solche durch Injektion oder als Infusion, z.B. in Kochsalzlösung, verabreicht werden können.

Zur oralen Verabreichung können die erfindungsgemäßen Komplexe in üblicher Weise mit üblichen Exzipienten zu Tabletten verpresst werden oder in Kapseln gefüllt werden.

Auch Präparate, die über einen längeren Zeitraum stabil sind, wie Tabletten (Kau-, Film-, Brausetabletten), Brausegranulate, Pulvermischungen, Sachets, in denen der Eisen(III)(II)-Komplex vorliegt sind beispielsweise geeignet.

Feste Einheitsdosierungsformen zur oralen Verabreichung enthalten beispielsweise 40 mg bis 120 mg, bevorzugter 60 mg bis 100 mg Eisen.

Vorzugsweise werden jedoch wässrige Lösungen oral verabreicht wie in der Form trinkbarer Formulierungen, wie Sirup, Elixier, Lösung, Suspension oder Saft.

Die erfindungsgemäßen Arzneimittel können ggf. weitere Bestandteile wie übliche pharmazeutische Träger- bzw. Hilfsstoffe, wie Bindemittel bzw. Gleitmittel, Verdünnungsmittel, Sprengmittel, Füllstoffe etc. enthalten. Tabletten können mit üblichen Filmbildnern beschichtet werden. Weiter können Aroma-, Geschmacks- und Farbstoffe zugesetzt werden, falls gewünscht.

Das erfindungsgemäße Arzneimittel kann ggf. auch weitere pharmakologisch wirksame Bestandteile enthalten, die aus der aus Vitaminen wie Ascorbinsäure, Spurenelementen, Mineralstoffen, Nährstoffen und Cofaktoren bestehenden Gruppe ausgewählt werden. Der/die weiteren pharmakologisch wirksamen Bestandteile sind bevorzugt die Vitamine ß-Carotin, Thiamin (Vitamin B₁), Riboflavin (Vitamin B₂), Pyridoxin (Vitamin B₆), Cyanocobalamin (Vitamin B₁₂), Cholecalciferol (Vitamin D₃), α-Tocopherol (Vitamin E), Biotin (Vitamin H), die Cofaktoren Pantothensäure, Nicotinamid, Folsäure, die Spurenelemente/Mineralien Kupfer, Mangan, Zink, Calcium, Phosphor und/oder Magnesium und die Nährstoffe Aminosäuren, Oligopeptide, Kohlenhydrate und Fette, gegebenenfalls in Form physiologisch verträglicher Salze. Als physiologisch verträgliche Salze kommen alle üblichen physiologisch verträglichen Salze in Frage, bevorzugt Salze anorganischer Säuren oder Basen wie Hydrochloride, Sulfate, Chloride, Phosphate, Hydrogenphosphate, Dihydrogenphosphate, Hydroxide oder Salze organischer Säuren wie z.B. Acetate, Fumarate, Maleate, Citrate usw. Die weiteren pharmakologische wirksamen Bestandteile können auch als Hydrate oder Solvate vorliegen. Phosphor wird bevorzugt in Form von Phosphaten oder Hydrogenphosphaten zugesetzt.

Die erfindungsgemäßen "mixed valence compounds" sind stabil und können gezielt Eisen(II) oder Eisen(III) an eine physiologische Umgebung abgeben. Ohne an eine Theorie gebunden zu sein, liegt in den erfindungsgemäßen Verbindungen polynukleares Eisenhydroxid, an das Kohlenhydrate, komplex und/oder über Wasserstoffbrückenbindungen gebunden sind, als eine Art chemische Matrix vor. In den erfindungsgemäßen Eisen-Kohlenhydrat-Komplex-Verbindungen liegt neben Eisen(III) auch Eisen(II) vor, welches jedoch in dieser Form überraschend eine verringerte Toxizität aufweist.

Die Mehrzahl der erfindungsgemäßen Eisen-Hydroxyd-Kohlenhydrat-Komplex-Verbindungen weist einen LD₅₀-Wert von etwa 200 mg Fe/kg Körpergewicht bis 600 mg Fe/kg Körpergewicht auf. Dieser LD₅₀-Wert wird intravenös bei Mäusen bestimmt. Im Vergleich dazu beträgt beispielsweise der LD₅₀ von Fe(II)-sulfat lediglich 11 mg Fe/kg Körpergewicht ebenfalls intravenös an Mäusen bestimmt (Berenbaum et al. 1960 zitiert in P. Geisser, M. Baer, E. Schraub: Arzneimittelforschung Drug Research 42 (II), 12, 1439-1452 (1992).

Gegenstand der Erfindung ist auch die Verwendung der erfindungsgemäßen Eisen(III)-Eisen(II)-Kohlenhydrat-Komplexe, wie in den Ansprüchen 1-8 definiert, zur Behandlung und Prophylaxe von Eisenmangelanämien bzw. zur Herstellung von Arzneimitteln zur Behandlung von Eisenmangelanämien. Die Arzneimittel sind zum Einsatz in der Human- und der Veterinärmedizin geeignet.

Die erfindungsgemäßen Eisen-Kohlenhydrat-Komplex-Verbindungen eignen sich somit auch zur Herstellung eines Medikamentes zur Behandlung von Patienten, die unter Symptomen einer Eisenmangelanämie leiden, wie zum Beispiel: Ermüdung, Antriebslosigkeit, Konzentrationschwäche, geringe kognitive Effizienz, Schwierigkeiten beim Finden der richtige Worte, Vergesslichkeit, unnatürliche Blässe, Reizbarkeit, Beschleunigung der Herzfrequenz (Tachykardie), wunde oder geschwollene Zunge, vergrößerte Milz, Schwangerengelüste (Pica), Kopfschmerzen, Appetitlosigkeit, erhöhte Infektionsanfälligkeit, depressive Verstimmungen.

Die erfindungsgemäßen Eisen-Kohlenhydrat-Komplex-Verbindungen eignen sich weiterhin zur Herstellung eines Medikamentes zur Behandlung der Eisenmangelanämie bei Schwangeren, der latenten Eisenmangelanämie bei Kindern und Heranwachsenden, der Eisenmangelanämie infolge gastrointestinaler Abnormalitäten, der Eisenmangelanämie infolge von Blutverlusten, wie durch gastrointestinale Blutungen (z.B. infolge von Geschwüren, Karzinomen, Hämorriden, entzündlichen Störungen, Einnahme von Acetylsalicylsäure), Menstruation, Verletzungen, Eisenmangelanämie infolge von Psilosis (sprue), Eisenmangelanämie infolge verminderter Eisenaufnahme bei der Ernährung, insbesondere bei selektiv essenden Kindern und Heranwachsenden, Immunschwäche hervorgerufen durch Eisenmangelanämie, Beeinträchtigung der Hirnleistung hervorgerufen durch Eisenmangelanämie, Restless Leg Syndrom.

Die erfindungsgemäß verwendeten Eisen-Kohlenhydrat-Komplex-Verbindung werden insbesondere oral oder parenteral verabreicht. Die tägliche Dosis beträgt beispielsweise zwischen 10 und 500 mg Eisen(III)/(II) proTag der Anwendung. Patienten mit Eisenmangel oder EisenmangelAnämie nehmen z.B. 2 bis 3 mal täglich je 100 mg Eisen(III)/(II) ein, und Schwangere 1 bis 2 mal täglich 60 mg Eisen(III)/(II) (jeweils berechnet als Eisen(III)/(II), nicht als Komplex).

Die Verabreichung kann bedenkenlos über einen Zeitraum von mehreren Monaten bis zur Verbesserung des Eisenstatus, reflektiert beispielsweise durch den Hämoglobin-Wert, die Transferrin-Sättigung und den Ferritin-Wert der Patienten, oder zur gewünschten Verbesserung einer durch Eisenmangelanämie hervorgerufenen Beeinträchtigung der Hirnleistung, der Immunantwort oder der Symptome des Restless Legs-Syndrom, erfolgen

Das erfindungsgemäße Präparat kann von Kindern, Jugendlichen und Erwachsenen eingenommen werden.

Die erfindungsgemäße Verwendung verläuft insbesondere mittels Verbesserung der Eisen-, Hämoglobin-, Ferritin- und Transferrinwerte, welche insbesondere bei Heranwachsenden und Kindern aber auch bei Erwachsenen mit einer Verbesserung im Kurzzeitgedächtnistests (STM), im Langzeitgedächtnistest (LTM), im Test der progressiven Matrices nach Raven, in der Welschers Erwachsenenintelligenzskala (WAIS) und/oder im emotionalen Koeffizienten (Baron EQ-i, YV-Test; Jugendversion), oder einer Verbesserung der Neutrophilen-Niveaus, der Antikörperniveaus und/oder der Lymphozytenfunktion einhergeht.

### Herstellungsbeispiele:

Die Ergebnisse der Beispiele 1 bis 4 sind in der Tabelle 1 zusammengefasst.

Die Prozentangaben beziehen sich auf Massenprozente.

### Beispiel 1

300 g Dextrin (DE-Wert 33) werden in 750 ml Wasser bei 60 °C gelöst. 341 g 12 % FeCl₃ und 444g 30 % NaOH werden innerhalb von 30 min bei 60 °C und einem konstanten pH-Wert von 11 ± 0.5 zudosiert. Die Reaktionslösung wird auf 100 °C aufgeheizt und 30 min bei dieser Temperatur gehalten. Die Reaktionslösung wird auf 25 °C abgekühlt und der pH-Wert mit 20 % HCl von 7.9 bis 8.0 eingestellt. Die Lösung wird 30 min bei 7000 U/min zentrifugiert und anschließend über einen AF-50 Filter filtriert, Das Produkt wird durch Zugabe von 92% Ethanol im Volumenverhältnis 1 : 2.4 (Reaktionslösung : Ethanol) ausgefällt und nach einer Absetzzeit von 1 Stunde isoliert. Das ölige Rohprodukt wird mit 92 % Ethanol gemixt bis ein Feststoff resultiert (2 × 200 ml), filtriert und anschließend für 16 Stunden bei 50 °C und 125 mbar getrocknet. Man erhält 126 g eines schwarzen, amorphen Pulvers,

### Beispiel 2

194 g Dextrin (DE-Wert 33) werden in 387 ml Wasser bei 60 °C gelöst. 176 g 12 % FeCl₃ und 229 g 30 % NaOH werden innerhalb von 30 min bei 60 °C und einem konstanten pH-Wert von 11 ± 0.5 zudosiert. Die Reaktionslösung wird auf 100 °C aufgeheizt und 30 min bei dieser Temperatur gehalten. Die Reaktionslösung wird auf 25 °C abgekühlt und der pH-Wert mit 30 % NaOH von 7.2 auf 8.0 eingestellt. Die Lösung wird über einen AF-50 Filter filtriert. Das Produkt wird durch Zugabe von 92% Ethanol im Volumenverhältnis 1 : 2.4 (Reaktionslösung : Ethanol) ausgefällt und nach einer Absetzzeit von 1 Stunde isoliert. Das ölige Rohprodukt wird mit 92 % Ethanol gemixt bis ein Festoff resultiert (4 x 200 ml), filtriert und anschließend für 16 Stunden bei 50 °C und 125 mbar getrocknet. Man erhält 75 g eines schwarzen, amorphen Pulvers.

### Beispiel 3

300 g Dextrin [DE-Wert 11) werden in 1200 ml Wasser bei 60 °C gelöst. 660 g 6.2 % FeCl₃ und 440 g 30 % NaOH werden innerhalb von 30 min bei 60 °C und einem konstanten pH-Wert von 11 ± 0.5 zudosiert. Die Reaktionslösung wird auf 100 °C aufgeheizt und 30 min bei dieser Temperatur gehalten. Die Reaktionslösung wird auf 25 °C abgekühlt und der pH-Wert mit 20 % HCl von 9.4 auf 8.0 eingestellt. Die Lösung wird 30 min bei 7000 U/min zentrifugiert und anschließend über einen AF-50 Filter filtriert. 1400 ml der Reaktionslösung werden durch Zugabe von 92% Ethanol im Volumenverhältnis 1 : 2.4 (Reaktionslösung : Ethanol) ausgefällt und nach einer Absetzzeit von 1 Stunde isoliert. Das ölige Rohprodukt wird mit 92 % Ethanol gemixt bis ein Feststoff resultiert (300 ml), filtriert und anschliessend für 16 Stunden bei 50 °C und 125 mbar getrocknet. Man erhält 50 g eines schwarzen, amorphen Pulvers.

### Beispiel 4

251 g Maltosesirup (wässrige Lösung 80 % - DE-Wert 39) werden in 1200 ml Wasser bei 60 °C gelöst. Der pH-Wert der Lösung wird mit 16 ml 30 % NaOH auf 11.0 eingestellt. 600 g 6.2 % FeCl₃ und 372 g 30 % NaOH werden innerhalb von 60 min bei 60 °C und einem konstanten pH-Wert von 11 ± 0.2 zudosiert. Die Reaktionslösung wird auf 100 °C aufgeheizt und 30 min bei dieser Temperatur gehalten, Die Reaktionslösung wird auf 25 °C abgekühlt und der pH-Wert mit 20 % HCl von 7.9 auf 6.0 eingestellt. Die Lösung wird über einen AF-50 Filter filtriert. Eine Hälfte der Reaktionslösung wird durch Zugabe von 92% Ethanol im Volumenverhältnis 1 :2.4 (Reaktionslösung : Ethanol) ausgefällt und das Rohprodukt nach einer Absetzzeit von 1 Stunde isoliert. Das ölige Rohprodukt wird mit 92 % Ethanol gemixt bis ein Feststoff resultiert (300 ml), filtriert und anschließend für 16 Stunden bei 50 °C und 125 mbar getrocknet, Man erhält 37 g eines schwarzen, amorphen Pulvers.

**Tabelle 1:**

| **Parameter** | Bsp. 1 | Bsp. 2 | Bsp. 3 | Bsp. 4 |
|---|---|---|---|---|
| Fe-Gehalt (%) | 20.7 | 16.9 | 18.1 | 25.9 |
| Fe(III)-Gehalt (%) | 15.5 | 11.7 | 16.7 | 22.1 |
| Fe(II)-Gehalt (%) | 5.2 | 5.2 | 1.4 | 3.8 |
| Fe(III)/Fe(II)-Verhältnis | 75 / 25 | 69 / 31 | 92 / 8 | 85 / 15 |
| NaCI-Gehalt (%) | 2.5 | 2.4 | 3.2 | 1.6 |
| Kohlenhydrat-Gehalt¹ (%) | 57 | 63 | 63 | 53 |
| pH | 8.3 | 8.7 | 8.4 | 6.3 |
| M_{w}² | 16000 | 18000 | 43000 | 16000 |
| Mₙ² | 10000 | 12000 | 18000 | 11000 |
| P³ | 1.6 | 1.5 | 2.5 | 1.4 |
| Fe-Ausbeute (%) | 64 | 60 | 33 | 52 |
| 1) Kohlenstoff-Gehalt bestimmt nach ASTM D5291 und auf Anhydroglucose berechnet. | | | | |
| 2) Bestimmt durch GPC gegen Pullulan als Standard | | | | |
| 3) P = Mw / Mn (Polydispersität) | | | | |

## Patentansprüche

1. Eisen-Kohlenhydrat-Komplex-Verbindung, die neben Eisen(III), Eisen(II) enthält, **dadurch gekennzeichnet, dass** der Anteil von Eisen(II) bezogen auf die Gesamtmenge des Eisens in der Komplex-Verbindung 2 bis 50 Gew.-% beträgt,
wobei die Kohlenhydrate ausgewählt werden aus unvollständig hydrolysierten Stärken, welche einen DE-Wert zwischen 0 und 100 aufweisen, Maltodextrinen mit einem DE-Wert von 3 bis 50, Maltosesirupen mit einem DE-Wert von 3 bis 50, Glukosesirupen mit einem DE-Wert von mehr als 50 und Cyclodextrinen.

2. Eisen-Kohlenhydrat-Komplex-Verbindung nach Anspruch 1, **dadurch gekennzeichnet, dass** der Anteil von Eisen(II) bezogen auf die Gesamtmenge des Eisens in der Komplex-Verbindung von 3 bis 50 Gew.-% beträgt.

3. Eisen-Kohlenhydrat-Komplex-Verbindung nach Anspruch 1 oder 2, **dadurch gekennzeichnet, dass** der Anteil des Kohlenhydrats (Kohlenstoff-Gehalt bestimmt nach ASTM D5291 und auf Anhydroglucose berechnet), bezogen auf die Eisen-Kohlenhydrat-Komplex-Verbindung 10 bis 80 Gew.-% beträgt.

4. Eisen-Kohlenhydrat-Komplex-Verbindung nach einem der Ansprüche 1 bis 3, **dadurch gekennzeichnet, dass** der Anteil des Eisens, bezogen auf die Eisen-Kohlenhydrat-Komplex-Verbindung 5 bis 40 Gew.-% beträgt.

5. Eisen-Kohlenhydrat-Komplex-Verbindung nach einem der Ansprüche 1 bis 4, **dadurch gekennzeichnet, dass** er
- 5 bis 40 Gew-% Eisen, wovon 3 bis 50 Gew.-% bezogen auf die Gesamt-Eisenmenge in der Form von Eisen(II) vorliegen,
- 10 bis 80 Gew.-% ein oder mehrere Kohlenhydrate (Kohlenstoff-Gehalt bestimmt nach ASTM D5291 und auf Anhydroglucose berechnet),
- Rest: Sauerstoff und Wasserstoff in gebundener Form (außer in Kohlenhydraten) sowie gegebenenfalls weitere Elemente
enthält.

6. Eisen-Kohlenhydrat-Komplex-Verbindung nach einem der Ansprüche 1 bis 5, **dadurch gekennzeichnet, dass** er
- 10 bis 30 Gew-% Eisen, wovon 5 bis 40 Gew.-% bezogen auf die Gesamt-Eisenmenge in der Form von Eisen(II) vorliegen,
- 20 bis 70 Gew.-% ein oder mehrere Kohlenhydrate (Kohlenstoff-Gehalt bestimmt nach ASTM D5291 und auf Anhydroglucose berechnet),
- Rest: Sauerstoff und Wasserstoff in gebundener Form (außer in Kohlenhydraten) sowie gegebenenfalls weitere Elemente enthält.

7. Eisen-Kohlenhydrat-Komplex-Verbindung nach einem der Ansprüche 1 bis 6, **dadurch gekennzeichnet, dass** ihr gewichtsmittleres Molekulargewicht 10 bis 80 kDa, bestimmt durch Gelpermeationschromatographie gegen Pullulan als Standard, beträgt.

8. Eisen-Kohlenhydrat-Komplex-Verbindung nach einem der Ansprüche 1 bis 7, **dadurch gekennzeichnet, dass** das Kohlenhydrat unvollständig hydrolysierte Stärke, welche einen DE-Wert zwischen 0 und 100 aufweist, ist.

9. Verfahren zur Herstellung einer Eisen-Kohlenhydrat-Komplex-Verbindung nach einem der vorstehenden Ansprüche, das die Schritte umfasst:
a) Herstellung einer wässrigen Lösung oder Suspension eines Kohlenhydrates, das die Fähigkeit besitzt, Eisen(III) zu Eisen (II) zu reduzieren, ausgewählt aus der Gruppe, die besteht aus: unvollständig hydrolysierten Stärken, welche einen DE-Wert zwischen 0 und 100 aufweisen, Maltodextrinen mit einem DE-Wert von 3 bis 50, Maltosesirupen mit einem DE-Wert von 3 bis 50, Glukosesirupen mit einem DE-Wert von mehr als 50,
b) Zugeben eines Eisen(III)-Salzes bei konstantem pH-Wert im Bereich von 7 - 13,
c) Erwärmen der wässrigen Lösung oder Suspension,
d) Abkühlen der wässrigen Lösung oder Suspension und
e) Isolieren der gebildeten Eisen-Kohlenhydrat-Komplex-Verbindung.

10. Verfahren zur Herstellung einer Eisen-Kohlenhydrat-Komplex-Verbindung nach einem der Ansprüche 1 bis 7, das die Schritte umfasst:
a) Herstellung einer wässrigen Lösung oder Suspension eines nicht reduzierenden Kohlenhydrates,
b) Zugeben eines Eisen(III)-Salzes im Gemisch mit einem Eisen(II)-Salz bei konstantem pH-Wert im Bereich von 7 - 13,
c) Erwärmen der wässrigen Lösung oder Suspension,
d) Abkühlen der wässrigen Lösung oder Suspension und
e) Isolieren der gebildeten Eisen-Kohlenhydrat-Komplex-Verbindung.

11. Verfahren zur Herstellung einer Eisen-Kohlenhydrat-Komplex-Verbindung nach einem der Ansprüche 1 bis 7, das die Schritte umfasst:
a) Herstellung einer wässrigen Lösung oder Suspension eines nicht reduzierenden Kohlenhydrates,
b) Zugeben eines Eisen(III)-Salzes und eines Reduktionsmittels, ausgewählt aus Vitamin C, Dihydroflavonen und Hydroperoxiden, vorzugsweise bei konstantem pH-Wert im Bereich von 7 - 13,
c) Erwärmen der wässrigen Lösung oder Suspension,
d) Abkühlen der wässrigen Lösung oder Suspension und
e) Isolieren der gebildeten Eisen-Kohlenhydrat-Komplex-Verbindung.

12. Verfahren nach Anspruch 9, **dadurch gekennzeichnet, dass** es nach dem Abkühlen der wässrigen Lösung oder Suspension in Schritt d) den Schritt d') der Einstellung des pH-Werts der wässrigen Lösung oder Suspension auf einen Wert zwischen 5 und 9 umfasst.

13. Verfahren nach Anspruch 9 oder 12, **dadurch gekennzeichnet, dass** das Eisen(III)-Salz ausgewählt wird aus der Gruppe, die besteht aus Eisen(III)-chlorid und Eisen(III)-sulfat.

14. Verfahren nach einem der Ansprüche 9, 12 oder 13, **dadurch gekennzeichnet, dass** in Schritt c) die wässrige Lösung oder Suspension auf mehr als 80 °C für mindestens 30 min erwärmt wird.

15. Verfahren nach einem der Ansprüche 9 oder 12 bis 13, **dadurch gekennzeichnet, dass** während der Zugabe des Eisen(III)-Salzes in Schritt b) und während des Erwärmens in Schritt c) der pH-Wert konstant gehalten wird.

16. Verfahren nach einem der Ansprüche 13 bis 15, **dadurch gekennzeichnet, dass** nach Schritt d') gegebenenfalls vorhandene Feststoffe abgetrennt werden, wonach die Eisen-Kohlenhydrat-Komplex-Verbindung aus der erhaltenen Lösung ausgefällt und abgetrennt wird.

17. Verfahren nach Anspruch 16, **dadurch gekennzeichnet, dass** die Abtrennung der Feststoffe durch Filtration oder Zentrifugation erfolgt.

18. Verfahren nach Anspruch 16 oder 17, **dadurch gekennzeichnet, dass** das Ausfällen der Eisen-Kohlenhydrat-Komplex-Verbindung mit einem oder mehreren Alkoholen, wie Ethanol oder Propanol erfolgt.

19. Verfahren nach mindestens einem der Ansprüche 16 bis 18, **dadurch gekennzeichnet, dass** die isolierte Eisen-Kohlenhydrat-Komplex-Verbindung anschließend mit Ethanol gemischt, filtriert und vakuumgetrocknet wird.

20. Arzneimittel, enthaltend eine Eisen-Kohlenhydrat-Komplex-Verbindung nach einem der Ansprüche 1 bis 8.

21. Eisen-Kohlenhydrat-Komplex-Verbindung nach einem der Ansprüche 1 bis 8 zur Verwendung als Arzneimittel.

22. Eisen-Kohlenhydrat-Komplex-Verbindung nach einem der Ansprüche 1 bis 8 zur Verwendung in der Behandlung von Eisenmangelanämien.

23. Eisen-Kohlenhydrat-Komplex-Verbindung nach Anspruch 21 oder 22 zur oralen oder parenteralen Verabreichung.

24. Eisen-Kohlenhydrat-Komplex-Verbindung nach einem der Ansprüche 21 bis 23, worin die verwendete Dosierungsform eine flüssige Formulierung ist, einschließlich einer trinkbaren Formulierung, wie ein Sirup, Elixier, Lösung, Suspension, Saft.

25. Eisen-Kohlenhydrat-Komplex-Verbindung nach einem der Ansprüche 21 bis 24, zur Herstellung eines Medikamentes zur Behandlung von Patienten, die unter Symptomen einer Eisenmangelanämie leiden.

26. Eisen-Kohlenhydrat-Komplex-Verbindung zur Verwendung nach Anspruch 25, worin die Symptome einschließen: Ermüdung, Antriebslosigkeit, Konzentrationsschwäche, geringe kognitive Effizienz, Schwierigkeiten beim Finden der richtige Worte, Vergesslichkeit, unnatürliche Blässe, Reizbarkeit, Beschleunigung der Herzfrequenz (Tachykardie), wunde oder geschwollene Zunge, vergrößerte Milz, Schwangerengelüste (Pica), Kopfschmerzen, Appetitlosigkeit, erhöhte Infektionsanfälligkeit, depressive Verstimmungen.

27. Eisen-Kohlenhydrat-Komplex-Verbindung nach einem der Ansprüche 21 bis 26 zur Herstellung eines Medikamentes zur Behandlung der Eisenmangelanämie bei Schwangeren, der latenten Eisenmangelanämie bei Kindern und Heranwachsenden, der Eisenmangelanämie infolge gastrointestinaler Abnormalitäten, der Eisenmangelanämie infolge von Blutverlusten, wie durch gastrointestinale Blutungen (z.B. infolge von Geschwüren, Karzinomen, Hämorriden, entzündlichen Störungen, Einnahme von Acetylsalicylsäure), Menstruation, Verletzungen, Eisenmangelanämie infolge von Psilosis (sprue), Eisenmangelanämie infolge verminderter Eisenaufnahme bei der Ernährung, insbesondere bei selektiv essenden Kindern und Heranwachsenden, Immunschwäche hervorgerufen durch Eisenmangelanämie, Beeinträchtigung der Hirnleistung hervorgerufen durch Eisenmangelanämie, Restless Legs Syndrom.

## Claims

1. An iron carbohydrate complex compound comprising iron(II) besides iron(III), **characterized in that** the proportion of iron(II) with regard to the overall amount of iron in the complex compound is 2 to 50 % by weight,
wherein the carbohydrates are selected from partially hydrolysed starches having a DE value comprised between 0 and 100, maltodextrins having a DE-value comprised between 3 and 50, maltose syrups having a DE-value comprised between 3 and 50, glucose syrups having a DE-value of more than 50 and cyclodextrins.

2. An iron carbohydrate complex compound according to claim1, **characterized in that** the proportion of iron(II) with regard to the overall amount of iron in the complex compound is comprised between 3 and 50 % by weight.

3. An iron carbohydrate complex compound according to claim 1 or 2, **characterized in that** the proportion of the carbohydrate (carbon content determined according to ASTM D5291 and calculated with respect to anhydroglucose) with regard to the iron carbohydrate complex compound is comprised between 10 and 80 % by weight.

4. An iron carbohydrate complex compound according to one of the claims 1 through 3, **characterized in that** the proportion of iron with regard to the iron carbohydrate complex compound is comprised between 5 and 40 % by weight.

5. An iron carbohydrate complex compound according to one of the claims 1 through 4, **characterized in that** it contains
- 5 through 40 % by weight iron, of which 3 through 50 % by weight with regard to the overall amount of iron are present in the form of iron(ii),
- 10 through 80 % by weight of one or more carbohydrates (carbon content determined according to ASTM D5291 and calculated with respect to anhydroglucose),
- rest: oxygen and hydrogen in bound form (except in carbohydrates) as well as, optionally, other elements.

6. An iron carbohydrate complex compound according to one of the claims 1 through 5, **characterized in that** it contains
- 10 through 30 % by weight iron, of which 5 through 40 % by weight with regard to the overall amount of iron are present in the form of iron(II),
- 20 through 70 % by weight of one or more carbohydrates (carbon content determined according to ASTM D5291 and calculated with respect to anhydroglucose),
- rest: oxygen and hydrogen in bound form (except in carbohydrates) as well as, optionally, other elements.

7. An iron carbohydrate complex compound according to one of the claims 1 through 6, **characterized in that** its weight average molecular weight is comprised between 10 and 80 kDa, determined by means of gel permeation chromatography against pullulan as standard.

8. An iron carbohydrate complex compound according to one of the claims 1 through 7, **characterized in that** the carbohydrate is partially hydrolysed starch having a DE value comprised between 0 and 100.

9. A method for producing an iron carbohydrate complex compound according to one of the preceding claims, the method comprising the steps of:
a) producing an aqueous solution or suspension of a carbohydrate which is able to reduce iron(III) to iron(II), selected from the group that consists of partially hydrolysed starches having a DE value comprised between 0 and 100, maltodextrins having a DE-value comprised between 3 and 50, maltose syrups having a DE-value comprised between 3 and 50, glucose syrups having a DE-value of more than 50,
b) adding an iron(III) salt at a constant pH value in the range of 7 - 13,
c) heating the aqueous solution or suspension,
d) cooling the aqueous solution or suspension and
e) isolating the formed iron carbohydrate complex compound.

10. A method for producing an iron carbohydrate complex compound according to one of the claims 1 through 7, the method comprising the steps of:
a) producing an aqueous solution or suspension of a non-reducing carbohydrate,
b) adding an iron(III) salt in mixture with an iron(II) salt at a constant pH value in the range of 7 - 13,
c) heating the aqueous solution or suspension,
d) cooling the aqueous solution or suspension and
e) isolating the formed iron carbohydrate complex compound.

11. A method for producing an iron carbohydrate complex compound according to one of the claims 1 through 7, the method comprising the steps of:
a) producing an aqueous solution or suspension of a non-reducing carbohydrate,
b) adding an iron(III) salt and a reducing agent selected from vitamin C, dihydroflavons and hydroperoxides, preferably at a constant pH value in the range of 7 - 13,
c) heating the aqueous solution or suspension,
d) cooling the aqueous solution or suspension and
e) isolating the formed iron carbohydrate complex compound.

12. A method according to claim 9, **characterized in that** it comprises, after the cooling of the aqueous solution or suspension in step d), the step d') of setting the pH value of the aqueous solution or suspension at a value comprised between 5 and 9.

13. A method according to claim 9 or 12, **characterized in that** the iron(III) salt is selected from the group which consists of iron(III) chloride and iron(III) sulphate.

14. A method according to one of the claims 9, 12 or 13, **characterized in that** in step c) the aqueous solution or suspension is heated up to more than 80°C for at least 30 min.

15. A method according to one of the claims 9 or 12 through 13, **characterized in that** during the addition of the iron(III) salt in step b) and during the heating in step c) the pH value is kept constant.

16. A method according to one of the claims 13 through 15, **characterized in that** after step d') possibly existing solids are separated, after which the iron carbohydrate complex compound is precipitated and separated from the obtained solution.

17. A method according to claim 16, **characterized in that** the separation of the solids is realized by means of filtration or centrifugation.

18. A method according to claim 16 or 17, **characterized in that** the precipitation of the iron carbohydrate complex compound is carried out by means of one or more alcohols, such as ethanol or propanol.

19. A method according to at least one of the claims 16 through 18, **characterized in that** the isolated iron carbohydrate complex compound is subsequently mixed with ethanol, filtrated and vacuum-dried.

20. A medicament containing an iron carbohydrate complex compound according to one of the claims 1 through 8.

21. An iron carbohydrate complex compound according to one of the claims 1 through 8, for the use as a medicament.

22. An iron carbohydrate complex compound according to one of the claims 1 through 8, for the use in the treatment of iron deficiency anaemias.

23. An iron carbohydrate complex compound according to claim 21 or 22 for oral or parenteral administration.

24. An iron carbohydrate complex compound according to one of the claims 21 through 23, wherein the used dosage form is a liquid formulation including a drinkable formulation, such as a syrup, an elixir, a solution, a suspension, a juice.

25. An iron carbohydrate complex compound according to one of the claims 21 through 24, for producing a medicament for the treatment of patients who suffer from symptoms of iron deficiency anaemia.

26. An iron carbohydrate complex compound for the use according to claim 25, wherein the symptoms include: tiredness, lethargy, lack of concentration, low cognitive efficiency, difficulties in finding the right words, obliviousness, unnatural paleness, irritability, acceleration of the heart rate (tachycardia), sore or swollen tongue, enlarged spleen, strong desires of pregnant women (pica), headaches, lack of appetite, increased susceptibility to infection, depressive moods.

27. An iron carbohydrate complex compound according to one of the claims 21 through 26, for producing a medicament for the treatment of iron deficiency anaemia in pregnant women, latent iron deficiency anaemia in children and adolescents, iron deficiency anaemia due to gastrointestinal abnormalities, iron deficiency anaemia due to losses of blood, such as caused by gastrointestinal bleedings (e.g. due to ulcers, carcinomas, haemorrhoids, inflammatory disorders, taking acetylsalicylic acid), menstruation, injuries, iron deficiency anaemia due to psilosis (sprue), iron deficiency anaemia due to reduced intake by nutrition, especially in selectively eating children and adolescents, immunodeficiency caused by iron deficiency anaemia, impairment of brain performance caused by iron deficiency anaemia, restless legs syndrome.

## Revendications

1. Composé de complexe fer-carbohydrate comprenant du fer(II) en plus due fer(III), **caractérisé en ce que** la proportion de fer(II) par rapport à la quantité totale du fer dans le composé de complexe est au moins 2 % en poids,
dans lequel les carbohydrates sont sélectionnés parmi des amidons partiellement hydrolysés ayant une valeur DE comprise entre 0 et 100, des maltodextrines ayant une valeur DE comprise entre 3 et 50, des sirops de maltose ayant une valeur DE comprise entre 3 et 50, des sirops de glucose ayant une valeur DE de plus de 50 et des cyclodextrines.

2. Composé de complexe fer-carbohydrate selon la revendication 1, **caractérisé en ce que** la proportion de fer(II) par rapport à la quantité totale du fer dans le composé de complexe est comprise entre 3 et 50 % en poids.

3. Composé de complexe fer-carbohydrate selon la revendication 1 ou la revendication 2, **caractérisé en ce que** la proportion du carbohydrate (la teneur en carbohydrate étant déterminée selon ASTM D5291 et calculée par rapport à anhydroglucose) par rapport au composé de complexe fer-carbohydrate est comprise entre 10 et 80 % en poids.

4. Composé de complexe fer-carbohydrate selon l'une des revendications 1 à 3, **caractérisé en ce que** la proportion du fer par rapport au composé de complexe fer-carbohydrate est comprise entre 5 et 40 % en poids.

5. Composé de complexe fer-carbohydrate selon l'une des revendications 1 à 4, **caractérisé en ce qu'**il contient
- 5 à 40 % en poids de fer, dont 3 à 50 % en poids par rapport à la quantité totale de fer sont présents en forme de fer(ii),
- 10 à 80 % en poids d'un ou de plusieurs carbohydrate(s) (la teneur en carbohydrate étant déterminée selon ASTM D5291 et calculée par rapport à anhydroglucose),
- le reste : de l'oxygène et de l'hydrogène sous forme liée (sauf dans les carbohydrates) ainsi que, le cas échéant, d'autre éléments.

6. Composé de complexe fer-carbohydrate selon l'une des revendications 1 à 5, **caractérisé en ce qu'**il contient
- 10 à 30 % en poids de fer, dont 5 à 40 % en poids par rapport à la quantité totale de fer sont présents en forme de fer(II),
- 20 à 70 % en poids d'un ou de plusieurs carbohydrate(s) (la teneur en carbohydrate étant déterminée selon ASTM D5291 et calculée par rapport à anhydroglucose),
- le reste : de l'oxygène et de l'hydrogène sous forme liée (sauf dans les carbohydrates) ainsi que, le cas échéant, d'autre éléments.

7. Composé de complexe fer-carbohydrate selon l'une des revendications 1 à 6, **caractérisé en ce que** son poids moléculaire moyen en poids est compris entre 10 et 80 kDa, déterminé à l'aide de la chromatographie par perméation de gel contre le pullulan comme standard.

8. Composé de complexe fer-carbohydrate selon l'une des revendications 1 à 7, **caractérisé en ce que** le carbohydrate est de l'amidon partiellement hydrolysé ayant une valeur DE compris entre 0 à 100.

9. Procédé de préparation d'un composé de complexe fer-carbohydrate selon l'une des revendications précédentes, le procédé comprenant les étapes de :
a) préparer une solution ou suspension aqueuse d'un carbohydrate, qui est capable de réduire du fer(III) au fer(II), sélectionné dans le groupe composé : des amidons partiellement hydrolysés ayant une valeur DE compris entre 0 et 100, des maltodextrines ayant une valeur DE comprise entre 3 et 50, des sirops de maltose ayant une valeur DE comprise entre 3 et 50, des sirops de glucose ayant une valeur DE de plus de 50,
b) ajouter un sel de fer(III) à une valeur de pH constante dans la gamme comprise entre 7 et 13,
c) chauffer la solution ou la suspension aqueuse,
d) refroidir la solution ou la suspension aqueuse et
e) isoler le composé de complexe fer-carbohydrate formé.

10. Procédé de préparation d'un composé de complexe fer-carbohydrate selon l'une des revendications 1 à 7, le procédé comprenant les étapes de :
a) préparer une solution ou suspension aqueuse d'un carbohydrate non-réduisant,
b) ajouter un sel de fer(III) dans un mélange avec un sel de fer(II) à une valeur de pH constante dans la gamme comprise entre 7 et 13,
c) chauffer la solution ou la suspension aqueuse,
d) refroidir la solution ou la suspension aqueuse et
e) isoler le composé de complexe fer-carbohydrate formé.

11. Procédé de préparation d'un composé de complexe fer-carbohydrate selon l'une des revendications 1 à 7, le procédé comprenant les étapes de :
a) préparer une solution ou suspension aqueuse d'un carbohydrate non-réduisant,
b) ajouter un sel de fer(III) et un agent réducteur sélectionné parmi la vitamine C, des dihydroflavons et des hydroperoxydes, de préférence à une valeur de pH constante dans la gamme comprise entre 7 et 13,
c) chauffer la solution ou la suspension aqueuse,
d) refroidir la solution ou la suspension aqueuse et
e) isoler le composé de complexe fer-carbohydrate formé.

12. Procédé selon la revendication 9, **caractérisé en ce qu'**il comprend, après le refroidissement de la solution ou de la suspension aqueuse dans l'étape d), une étape d') pour fixer la valeur de pH de la solution ou de la suspension aqueuse à une valeur comprise entre 5 et 9.

13. Procédé selon la revendication 9 ou la revendication 12, **caractérisé en ce que** le sel de fer(III) est sélectionné dans le groupe que est composé de la chlorure de fer(III) et du sulfate de fer(III).

14. Procédé selon l'une des revendications 9, 12 ou 13, **caractérisé en ce que** dans l'étape c) la solution ou la suspension aqueuse est chauffée à plus de 80°C pour au moins 30 min.

15. Procédé selon l'une des revendications 9 ou 12 à 13, **caractérisé en ce que** pendant l'addition du sel de fer(III) dans l'étape b) et pendant le réchauffement dans l'étape c) la valeur de pH est maintenue constante.

16. Procédé selon l'une des revendications 13 à 15, **caractérisé en ce qu'**après l'étape d') des solides, qui sont, le cas échéant, présents, sont séparés, à la suite de quoi le composé de complexe fer-carbohydrate est précipité et séparé de la solution obtenue.

17. Procédé selon la revendication 16, **caractérisé en ce que** la séparation des solides se fait par la filtration ou par la centrifugation.

18. Procédé selon la revendication 16 ou la revendication 17, **caractérisé en ce que** la précipitation du composé de complexe fer-carbohydrate est effectuée par moyen d'un ou de plusieurs alcool(s), tels que l'éthanol ou le propanol.

19. Procédé selon l'au moins une des revendications 16 à 18, **caractérisé en ce que** le composé de complexe fer-carbohydrate isolé est ensuite mélangé avec de l'éthanol, filtré et séché sous vide.

20. Médicament contenant un composé de complexe fer-carbohydrate selon l'une des revendications 1 à 8.

21. Composé de complexe fer-carbohydrate selon l'une des revendications 1 à 8 pour l'utilisation comme médicament.

22. Composé de complexe fer-carbohydrate selon l'une des revendications 1 à 8 pour l'utilisation dans le traitement des anémies ferriprives.

23. Composé de complexe fer-carbohydrate selon la revendication 21 ou la revendication 22 pour l'administration orale ou parentérale.

24. Composé de complexe fer-carbohydrate selon l'une des revendications 21 à 23, dans lequel la forme de dosage utilisée est une formulation liquide, une formulation potable y compris, telle qu'un sirop, un élixir, une solution, une suspension, un jus.

25. Composé de complexe fer-carbohydrate selon l'une des revendications 21 à 24, pour la préparation d'un médicament destiné au traitement de patients qui souffrent des symptômes d'une anémie ferriprive.

26. Composé de complexe fer-carbohydrate pour l'utilisation selon la revendication 25, dans lequel les symptômes comprennent : la fatigue, la léthargie, le manque de concentration, une efficience cognitive faible, des difficultés de trouver les bons mots, les troubles de mémoire, la pâleur non-naturelle, l'irritabilité, l'accélération de la fréquence cardiaque (tachycardie), une langue endolorie ou gonflée, une rate dilatée, des désirs des femmes enceintes (pica), des maux de tête, le manque d'appétit, une susceptibilité accrue aux infections, des états dépressifs.

27. Composé de complexe fer-carbohydrate selon l'une des revendications 21 à 26, pour la préparation d'un médicament destiné au traitement de l'anémie ferriprive chez les femmes enceintes, de l'anémie ferriprive latente chez les enfants et les adolescents, de l'anémie ferriprive causée par des anormalités gastro-intestinales, de l'anémie ferriprive causée par des pertes de sang, telles que des saignements gastro-intestinaux (par exemple en raison des ulcères, des carcinomes, des hémorroïdes, des troubles inflammatoires, de l'ingestion de l'acide acétylsalicylique), de la menstruation, des blessures, de l'anémie ferriprive par suite de la psilosis (sprue), de l'anémie ferriprive par suite d'un apport en fer réduit dans l'alimentation, notamment chez des enfants et des adolescents qui mangent sélectivement, de l'immunodéficience causée par l'anémie ferriprive, de la déficience de la performance cérébrale causée par l'anémie ferriprive, du syndrome des jambes sans repos.
